# EUROPÄISCHE PATENTANMELDUNG

(11) **EP 2 062 607 A1**
(43) Veröffentlichungstag der Anmeldung: **27.05.2009**
(21) Anmeldenummer: 08015239.0
(22) Anmeldetag: 28.08.2008
(51) Int. Cl.: A61M 5/24, A61M 5/31

(54) **Spritze mit Haftbeschichtung im Griffbereich**

(30) Priorität: 22.11.2007 DE 102007056240
(71) Anmelder: Henke-Sass, Wolf GmbH, 78532 Tuttlingen (DE)
(72) Erfinder: Rückert, Ralf, 78573 Wurmlingen (DE); Knefel, Stefan, 78256 Steisslingen (DE)
(74) Vertreter: Geyer, Fehners & Partner

(57) **Zusammenfassung**

Es wird eine Spritze mit einem Spritzenkörper (2), einer Kolbenstange (3), die im Spritzenkörper (2) längsverschieblich geführt ist, und einem Griffbereich (23) mit einem am Spritzenkörper (2) ausgebildeten Fingerabschnitt (10) und einem am hinteren Ende der Kolbenstange (3) ausgebildeten Daumenabschnitt (17) bereitgestellt, wobei der Griffbereich (23) zumindest teilweise mit einer Reibauflage (18, 19) versehen ist, die aus einem anderen Material besteht als der Griffbereich (23).

## Beschreibung

Die vorliegende Erfindung betrifft eine Spritze mit einem Spritzenkörper, einer Kolbenstange, die im Spritzenkörper längsverschieblich geführt ist, und einem Griffbereich mit einem am Spritzenkörper ausgebildeten Fingerabschnitt und einem am hinteren Ende der Kolbenstange ausgebildeten Daumenabschnitt.

Solche Spritzen sind häufig, falls sie wiederverwendbare Spritzen sind, aus Metall hergestellt. Die Metalloberfläche ist in der Regel möglichst glatt, um die Spritze gut sterilisieren bzw. autoklavieren zu können. Dies führt jedoch zu der Schwierigkeit, daß man bei der Bedienung der Spritze relativ leicht mit den Fingern vom Fingerabschnitt oder mit dem Daumen vom Daumenabschnitt abrutschen kann.

Ausgehend hiervon ist es Aufgabe der Erfindung, eine Spritze der eingangs genannten Art so weiterzubilden, daß eine einfache und sichere Bedienung der Spritze gewährleistet werden kann.

Die Aufgabe wird bei einer Spritze der eingangs genannten Art dadurch gelöst, daß der Griffbereich zumindest teilweise mit einer Reibauflage versehen ist, die aus einem anderen Material besteht als der Griffbereich. Mit dieser Reibauflage wird der Vorteil erreicht, daß eine höhere Reibung zwischen dem Finger bzw. Daumen, der auf der Reibauflage aufliegt, vorliegt und somit sicher ein Abrutschen vermieden werden kann.

Insbesondere ist der Griffbereich zumindest teilweise mit der Reibauflage ummantelt. Dies ist eine besonders einfache Art, um im Griffbereich die Reibauflage so vorzusehen, daß sie selbst nicht gegenüber dem Griffbereich verrutschen kann. Natürlich kann der gesamte Griffbereich mit der Reibauflage ummantelt sein.

Die Spritze kann als wiederverwendbare Spritze ausgebildet sein, wobei der Spritzenkörper und der Griffbereich im wesentlichen aus Metall hergestellt sind. Es ist insbesondere möglich, daß der Spritzenkörper und der Griffbereich vollständig aus Metall hergestellt sind. Eine solche wiederverwendbare Spritze ist sehr lange einsetzbar.

Die Reibauflage kann aus Kunststoff hergestellt sein. Insbesondere kann sie aus Silikon bestehen. So kann selbsthaftendes Silikon verwendet werden.

Das Material der Reibauflage wird bevorzugt so gewählt, daß die Spritze weiterhin autoklavierbar ist und somit sehr gut sterilisiert werden kann.

Unter autoklavierbar wird hier insbesondere verstanden, daß die Spritze mindestens mehrere Minuten gesättigtem Wasserdampf von 120 - 140°C zur Sterilisation ausgesetzt werden kann, ohne daß dabei die Spritze und insbesondere die Reibauflage beschädigt wird.

Die Reibauflage ist insbesondere aus einem Material, das weicher ist als das Material des Griffbereiches. Damit wird durch ein leichtes Einsinken der Finger bzw. des Daumens beim Kontakt mit der Reibauflage zusätzlich ein Abrutschen verhindert.

Der Daumenabschnitt kann ringförmig ausgebildet sein. Insbesondere kann er kreisringförmig oder oval ausgebildet sein. Die kreisringförmige Ausbildung wird bevorzugt für männliche Benutzer gewählt, wohingegen die ovale Ausbildung des Daumenabschnitts bevorzugt für weibliche Benutzer bereitgestellt wird.

Die Dicke der Reibauflage ist bevorzugt konstant. Natürlich ist es auch möglich, die Dicke zu variieren und dadurch z.B. vorgeformte Mulden für den bzw. die Finger oder den Daumen bereitzustellen.

Die Reibauflage kann insbesondere durch ein Spritzgußverfahren im Griffbereich angebracht werden.

Die Spritze kann insbesondere als Karpulen-Spritze ausgebildet werden. Eine Karpulen-Spritze wird häufig im zahnmedizinischen Bereich zur Lokalanästhesie eingesetzt. In diesem Fall ist es möglich, die sogenannte Aspirationsprobe durchzuführen, bei der nach dem Einstich der Nadel der Spritze in den zu betäubenden Bereich leicht angesaugt wird (durch Zurückziehen der Kolbenstange mittels des Daumenabschnitts) um sicherzustellen, daß das Medikament zur Lokalanästhesie in den richtigen Bereich eingespritzt wird.

Bei der Spritze kann die Reibauflage eingefärbt sein, wobei die Farbe einen Parameter der Spritze kodiert. Insbesondere kann durch diese Farbkodierung eine Zuordnung zu einem Bediener realisiert werden. Auch ist es möglich, den Einsatzbereich der Spritze zu kodieren.

Die erfindungsgemäße Spritze ist bevorzugt eine medizinische Spritze. Insbesondere handelt es sich um eine Humanmedizin-Spritze, z.B. eine Dentalmedizin-Spritze. Sie kann jedoch auch als Veterinärmedizin-Spritze ausgebildet sein.

Ferner kann noch ein Satz der erfindungsgemäßen Spritzen bereitgestellt werden, wobei sich die Spritzen des Satzes bevorzugt nur durch die Farbe der Reibauflage unterscheiden. Damit wird eine Farbkodierung der sonst identischen Spritzen erreicht, so daß ein unerwünschtes Vertauschen der einzelnen Spritzen des Satzes sicher vermieden werden kann.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine perspektivische Darstellung einer ersten Ausführungsform der erfindungsgemäßen Spritze;
- Fig. 2: eine Schnittdarstellung der Spritze von Fig. 1;
- Fig. 3: eine schematische Darstellung einer Zylinderampulle 13;
- Fig. 4: eine perspektivische Darstellung einer zweiten Ausführungsform der erfindungsgemäßen Spritze, und
- Fig. 5: eine Schnittdarstellung der Spritze von Fig. 4.

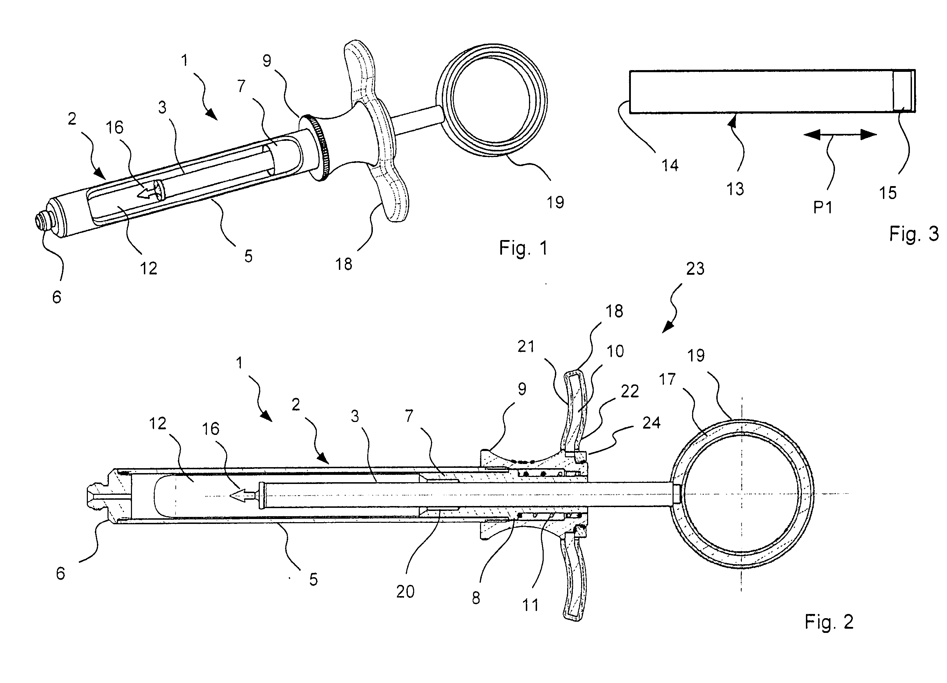

Bei der in den Figuren 1 und 2 gezeigten Ausführungsformen ist die erfindungsgemäße Spritze 1 als Karpulenspritze, die auch als Zylinderampullenspritze bezeichnet werden kann, ausgebildet. Die Spritze 1 umfaßt einen im wesentlichen rohrförmig ausgebildeten Spritzenkörper 2, in dem eine Kolbenstange 3 längsverschieblich gelagert ist.

Der Spritzenkörper 2 umfaßt ein hohlzylinderförmiges Hauptrohr 5 mit einer Nadelaufnahme 6 am vorderen Ende und eine am hinteren Ende eingesetzte Zentrierhülse 7. Die Nadelaufnahme 6 ist in das Hauptrohr 5 eingeschraubt und verklebt. Die Zentrierhülse 7 ist bis zu ihrem Anschlag 8, der am hinteren Ende des Hauptrohrs 5 anliegt, in das Hauptrohr 5 eingeführt. Ferner ist am hinteren Ende des Hauptrohres 5 eine Griffkappe 9 aufgeschraubt und verklebt, auf deren hinterem Ende eine Fingerstütze 10 drehbar gelagert und durch einen Ring 24 gesichert ist. Der Ring 24 ist dazu auf dem hinteren Ende der Fingerstütze 10 aufgeschraubt und verklebt.

Zwischen der Zentrierhülse 7 und der Griffkappe 9 ist eine Spiralfeder 11 angeordnet, deren Enden mit der Zentrierhülse 7 und der Griffkappe 9 verbunden sind, so daß die Zentrierhülse 7 gegen das Hauptrohr 5 gedrückt wird.

Das Hauptrohr 5 weist eine seitliche Beladungsöffnung 12 auf, über die die Spritze 1 mit einer schematisch in Fig. 3 gezeigten Zylinderampulle 13 beladen werden kann. Bei der Zylinderampulle 13 kann es sich um eine herkömmliche Glasampulle handeln, die an ihrem vorderen Ende mit einer Membranschicht 14 und an ihrem hinteren Ende mit einem in Längsrichtung der Zylinderampulle 13 verschiebbaren (wie durch den Doppelpfeil P1 angedeutet ist) Gummistopfen 15 verschlossen ist.

Die Kolbenstange 3 weist an ihrem vorderen Ende eine Pfeilspitze 16 auf, die in den Gummistopfen 15 der eingelegten Zylinderampulle 13 so eingestoßen werden kann, daß der Gummistopfen 15 durch Bewegung der Kolbenstange 3 in Längsrichtung verschoben werden kann. Die Pfeilspitze 16 kann somit im Gummistopfen 15 verankert werden.

Am hinteren Ende der Kolbenstange 3 ist ein kreisringförmiger Ziehring 17 für den Daumen eines Benutzers der Spritze 1 befestigt. Die bisher beschriebenen Teile der Spritze 1 sind alle aus Metall hergestellt, wobei die Kolbenstange 3 aus Edelstahl besteht und die restlichen Teile (bis auf die Druckfeder 11) aus verchromten Messing hergestellt sind.

Der Spritzenkörper 2 weist eine Länge von ca. 110 mm auf und der Innendurchmesser des Hauptrohrs beträgt ca. 10 mm. Die Kolbenstange 3 weist eine Länge von ca. 100 mm auf. Der Außen- und Innendurchmesser des Ziehrings 17 in Fig. 2 beträgt ca. 32 bzw. 30 mm.

Um einem Bediener die Benutzung der Spritze 1 zu erleichtern, sind sowohl die Fingerstütze 10 (im folgenden auch Fingerabschnitt genannt) als auch der Ziehring 17 (im folgenden auch Daumenabschnitt 17 genannt) mit einer Schicht 18 bzw. 19 aus selbsthaftendem Silikon ummantelt. Dies führt zu dem Vorteil, daß zwischen den Fingern des Benutzers und dem Fingerabschnitt 10 sowie dem Daumen des Benutzers und dem Daumenabschnitt 17 eine höhere Reibung vorliegt verglichen mit dem Fall, bei dem keine Silikonummantelung 18, 19 vorgesehen ist und somit die Finger direkt mit dem Fingerabschnitt 10 und der Daumen direkt mit dem Daumenabschnitt 17 in Kontakt stehen. Diese erhöhte Reibung erleichtert die Handhabung der Spritze 1. Die Dicke der Schichten 18 und 19 ist bevorzugt möglichst konstant. In dem hier beschriebenen Ausführungsbeispiel liegt sie in einem Bereich von 0,5 - 1 mm.

Im Betrieb wird die Kolbenstange 3 mittels dem Daumenabschnitt 17 soweit nach hinten gezogen, bis die Pfeilspitze 16 vollständig in einem Aufnahmebereich 20 am vorderen Ende der Zentrierhülse 7 versenkt ist. Ein weiteres Zurückziehen der Kolbenstange 3 führt dann dazu, daß die Zentrierhülse 7 in Längsrichtung des Hauptrohrs 5 gegen die Feder 11 verschoben wird, so daß ausreichend Platz im Hauptrohr zum Einlegen der Zylinderampulle 13 vorliegt. In dieser Stellung wird die Kolbenstange 3 während des Einsetzens der Zylinderampulle 13 gehalten. Sobald die Zylinderampulle 13 über die seitliche Beladungsöffnung 2 in das Hauptrohr 5 eingesetzt wurde, kann das Halten der Stellung der Kolbenstange 3 beendet werden, so daß aufgrund der Rückstellkraft der Feder 11 die Zentrierhülse 7 die eingelegte Zylinderampulle 13 gegen die Nadelaufnahme 6 drückt und somit die Zylinderampulle 13 im Hauptrohr 5 fixiert.

Dann wird die Kolbenstange 3 nach vorne geschoben, um die Pfeilspitze 16 in den Gummistopfen 15 einzustechen. Ferner wird durch die Nadelaufnahme 6 und die Membran 14 eine Nadel (nicht gezeigt) in die Zylinderampulle 13 eingestochen. Die Spritze 1 ist nun für den Einsatz vorbereitet. Aufgrund der Ummantelungen 18 und 19 wird ein Abrutschen der Finger vom Fingerabschnitt 10 (von seiner vorderen bzw. hinteren Seite 21, 22, je nachdem, wie die Kolbenstange 3 bewegt wird) sowie ein Herausrutschen des Daumens aus dem Daumenabschnitt 17 verhindert.

Die Fingerstütze 10 und der Ziehring 17 bilden einen Griffbereich 23 der Spritze 1. Der Griffbereich 23 ist hierbei so ausgebildet, daß die Spritze 1, wenn eine Zylinderampulle 13 eingesetzt ist, mit einer Hand bedient werden kann. Insbesondere ist eine einhändige Bedienung derart möglich, daß die Kolbenstange 3 zum vorderen Ende des Hauptrohrs 5 hin und von diesem vorderen Ende weg in Längsrichtung des Hauptrohrs 5 bewegt werden kann.

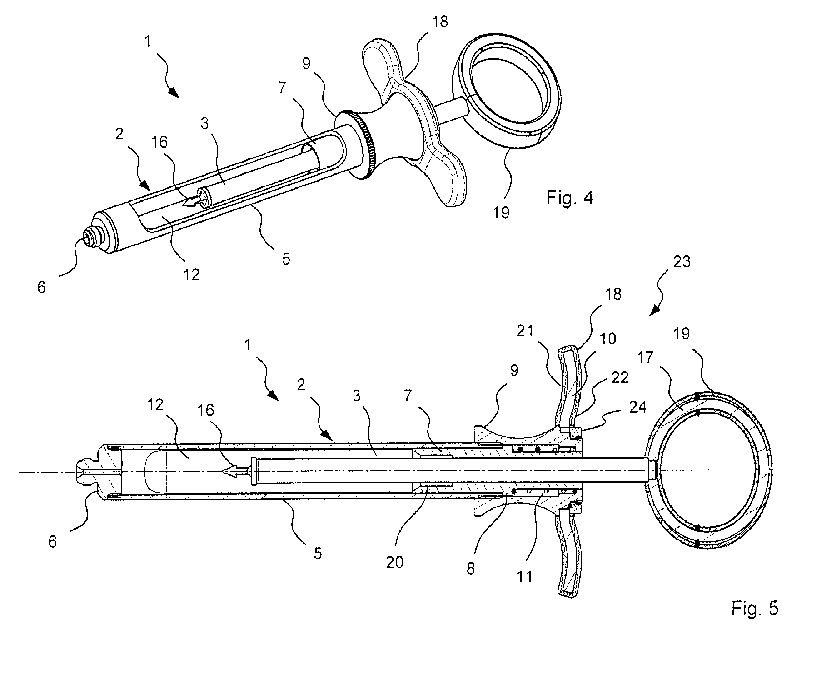

In den Figuren 4 und 5 ist eine Abwandlung der Spritze 1 von Fig. 1 und 2 gezeigt. Gleiche Elemente werden mit gleichen Bezugszeichen bezeichnet und zu deren Beschreibung wird auf die obigen Ausführungen verwiesen.

Die Spritze 1 gemäß Figuren 4 und 5 unterscheidet sich von der Spritze 1, die in den Figuren 1 und 2 gezeigt ist, durch die Ausbildung des Daumenabschnittes 17. Bei der Spritze von Fig. 4 und 5 ist der Daumenabschnitt 17 oval ausgebildet und nicht mehr kreisringförmig. Ferner ist die Länge der Kolbenstande 3 etwas geringer. Jedoch ist auch der ovale Daumenabschnitt 17 mit einer Silikonschicht 19 ummantelt.

Die Ummantelungen 18 und 19 können eine glatte Oberfläche aufweisen. Es ist jedoch auch möglich, die Oberflächenstruktur anders zu wählen. Insbesondere sind die Ummantelungen 18 und 19 aus einem weicheren Material als das Material, aus dem der Fingerabschnitt 10 bzw. der Daumenabschnitt 17 hergestellt ist.

Es können mehrere gleich ausgebildete Spritzen 1 vorgesehen werden, die sich nur durch die Farbe der Ummantelungen 18 und 19 unterscheiden. Damit wird noch zusätzlich der Vorteil erreicht, daß die einzelnen Spritzen leicht zu unterscheiden sind, so daß man z.B. eine Spritze einem speziellen Bediener leicht zuordnen kann.

## Patentansprüche

1. Spritze mit
einem Spritzenkörper (2),
einer Kolbenstange (3), die im Spritzenkörper (2) längsverschieblich geführt ist,
und einem Griffbereich (23) mit einem am Spritzenkörper (2) ausgebildeten Fingerabschnitt (10) und einem am hinteren Ende der Kolbenstange (3) ausgebildeten Daumenabschnitt (17),
**dadurch gekennzeichnet, daß**
der Griffbereich (23) zumindest teilweise mit einer Reibauflage (18, 19) versehen ist, die aus einem anderen Material besteht als der Griffbereich (23).

2. Spritze nach Anspruch 1, **dadurch gekennzeichnet, daß** der Griffbereich (23) zumindest teilweise mit der Reibauflage (18, 19) ummantelt ist.

3. Spritze nach einem der obigen Ansprüche, **dadurch gekennzeichnet, daß** die Spritze als wiederverwendbare Spritze ausgebildet ist, wobei der Spritzenkörper (2) und der Griffbereich (23) im wesentlichen aus Metall hergestellt sind.

4. Spritze nach einem der obigen Ansprüche, **dadurch gekennzeichnet, daß** die Reibauflage (18, 19) aus Kunststoff, insbesondere aus Silikon hergestellt ist.

5. Spritze nach einem der obigen Ansprüche, **dadurch gekennzeichnet, daß** die Reibauflage (18, 19) weicher als der Griffbereich (23) ist.

6. Spritze nach einem der obigen Ansprüche, **dadurch gekennzeichnet, daß** der Daumenabschnitt (17) ringförmig ausgebildet ist.

7. Spritze nach Anspruch 6, **dadurch gekennzeichnet, daß** der Daumenabschnitt (17) kreisringförmig oder oval ausgebildet ist.

8. Spritze nach einem der obigen Ansprüche, **dadurch gekennzeichnet, daß** die Reibauflage (18, 19) konstante Dicke aufweist.

9. Spritze nach einem der obigen Ansprüche, **dadurch gekennzeichnet, daß** die Spritze als Karpulen-Spritze ausgebildet ist.

10. Spritze nach einem der obigen Ansprüche, **dadurch gekennzeichnet, daß** die Reibauflage (18, 19) eingefärbt ist, wobei die Farbe einen Parameter der Spritze kodiert.

11. Satz von Spritzen nach Anspruch 10, **dadurch gekennzeichnet, daß** sich die Spritzen (1) nur durch die Farbe der Reibauflage (18, 19) unterscheiden.
